Europäisches Patentamt

European Patent Office

Office européen des brevets

(11) Publication number: **0 124 599**
**B1**

(12) **EUROPEAN PATENT SPECIFICATION**

(45) Date of publication of patent specification: 02.09.87

(21) Application number: **83903854.4**

(22) Date of filing: **28.10.83**

(86) International application number:
**PCT/US83/01709**

(87) International publication number:
**WO 84/01805 10.05.84 Gazette 84/12**

(51) Int. Cl.⁴: **F 16 K 11/07, A 61 B 17/00**

(54) **MEDICAL STOPCOCK VALVE ASSEMBLY.**

(30) Priority: **01.11.82 US 438410**

(43) Date of publication of application:
**14.11.84 Bulletin 84/46**

(45) Publication of the grant of the patent:
**02.09.87 Bulletin 87/36**

(84) Designated Contracting States:
**AT BE CH DE FR GB LI LU NL SE**

(56) References cited:
**US-A-2 854 027**
**US-A-3 157 201**
**US-A-3 185 179**
**US-A-3 186 437**
**US-A-3 344 785**
**US-A-3 773 604**
**US-A-3 783 900**
**US-A-3 834 372**
**US-A-3 957 082**
**US-A-3 987 930**
**US-A-4 003 403**
**US-A-4 112 932**
**US-A-4 207 923**
**US-A-4 219 021**

(73) Proprietor: **PARHAM, Allan M.**
**302 Plantation Drive**
**New Bern, NC 28560 (US)**

(72) Inventor: **PARHAM, Allan M.**
**302 Plantation Drive**
**New Bern, NC 28560 (US)**

(74) Representative: **Charrier, Rolf, Dipl.-Ing.**
**Postfach 260 Rehlingenstrasse 8**
**D-8900 Augsburg 31 (DE)**

Courier Press, Leamington Spa, England.

EP 0 124 599 B1

## Description

The present invention relates to a fluid conveying member for use in a medical fluid flow system according to the preamble of claim 1.

Fluid conveying members such as small plastic stopcock valve assemblies have long been used routinely in a variety of medical procedures and operations such as, for example, metering the infusion of fluids into the bodies of persons under medical care. Typically, such stopcock valve assemblies include at least a three-way type valve wherein the valve body has three or more radially-arranged ports, and an interior directional control member is rotatably arranged within the valve body for selectively closing one or more ports while opening communication between at least two other ports.

Examples of this type of valve are discussed in US—A—3 774 604 including a valve body having formed therein a plurality of fluid ports, an operating member in said valve body for selectively controlling fluid communication through said valve body between said fluid ports, and at least one closure means having engagement means formed for selective engagement with said valve body at at least one of said fluid ports for closure thereof, comprising closure-receiving means formed integrally on said valve body having engagement means corresponding to the engagement means formed on the closure means to receive and retain said closure means.

As will of course be understood, the maintenance of sterile conditions in such uses of these fluid conveying members is exceedingly important. Accordingly, when one of the ports is closed and out of use, a cap or other similar closure device is ordinarily fitted in the port to prevent possible entrance therethrough of bacteria, germs or the like into the valve body. In the typical use of such fluid conveying members it is often desirable or necessary to alter the operational disposition of the fluid conveying member to bring into operation the out-of-use port which of course requires the removal of the cap therefrom. For example, in infusion usage of a valve, it is routine procedure to periodically sample and test the infusion fluid flowing through the valve and/or the body fluid to maintain close control thereof, this being readily accomplished by removing the cap from the out-of-use port and rotating the directional control member to divert a small quantity of the infusion fluid or to withdraw a small quantity of the body fluid through the out-of-use port. Ordinarily any such procedure requires the attendant performing it to use both hands necessitating the cap be placed aside and, in practice, the cap, which is normally very small, is sometimes lost or temporarily misplaced, all of which can create particular problems when the valve is to be returned to its original operational disposition and the cap is to be replaced. As will be understood, the mere setting aside of the cap in itself can jeopardize the sterility thereof and of the valve upon replacement of the cap and, if the procedure requires any significant amount of time to complete, it is ordinary practice to discard the removed caps and to replace it with a new cap when the valve is returned to its original operating state. However, additionally, the loss or misplacement of the cap necessitates that the valve, upon return to its original operating state, be operated at least temporarily without a cap on the out-of-use port until the lost or misplaced cap is located or a replacement cap is obtained, which poses a more significant problem in maintaining the desired sterile condition of the valve.

The present invention provides a significant improvement over the above-described conventional fluid conveying members such as stopcock valves by providing a blind accessory member on the fluid conveying member adapted to receive and store one or more closure means when out of use to prevent loss thereof and to provide ready access thereto for use when desired.

The present invention provides improvement in medical fluid conveying members of the type comprising a tubular portion having formed therein a fluid conveying passage way and having connection means at its ends for engagement with other fluid conveying members, with at least one closure means for port closing engagement with a medical stopcock valve. Briefly described, the improvement includes a blind portion of said tubular portion out of communication with said passage way formed for engagement by said closure means to receive and retain it for storage when not in port-closing use.

The fluid conveying member of the present invention is a storage member for the port closure means. The port closure means preferably comprises a cap.

Preferably, removeably attached to said tubular portion is a unit having a plurality of cap-receiving means adapted for a stored engagement of plural caps. In an embodiment the tubular portion is one of the tubes of the stopcock valve assembly and said tube and said cap storage unit are formed with mating elements permitting sliding receipt of the cap storage unit on the tube for ready access to its caps for use and permitting sliding removal of the cap storage unit from the tube following use of the stored caps for replenishment of the cap storage unit with other caps or for replacement by another cap storage unit having other caps stored thereon.

Brief Description of the Drawings

Figure 1 is a perspective view of a medical stopcock valve assembly according to the preferred embodiment of the present invention;

Figure 2 is a horizontal cross-sectional view thereof taken along line 2—2 of Figure 1;

Figure 3 is a perspective view of a modified form of medical stopcock valve assembly according to another embodiment of the present invention;

Figure 4 is a perspective view of a modified form of cap according to another embodiment of the present invention;

Figure 5 is a perspective view of a medical stopcock valve assembly employing the modified cap of Figure 4;

Figure 6 is a perspective view of a modified form of medical stopcock valve assembly according to another embodiment of the present invention; and

Figure 7 is a perspective view of a cap storage member of the type of Figure 6 illustrating three alternate arrangements for stored mounting of caps according to further embodiments of the present invention.

## Description of the Preferred Embodiment

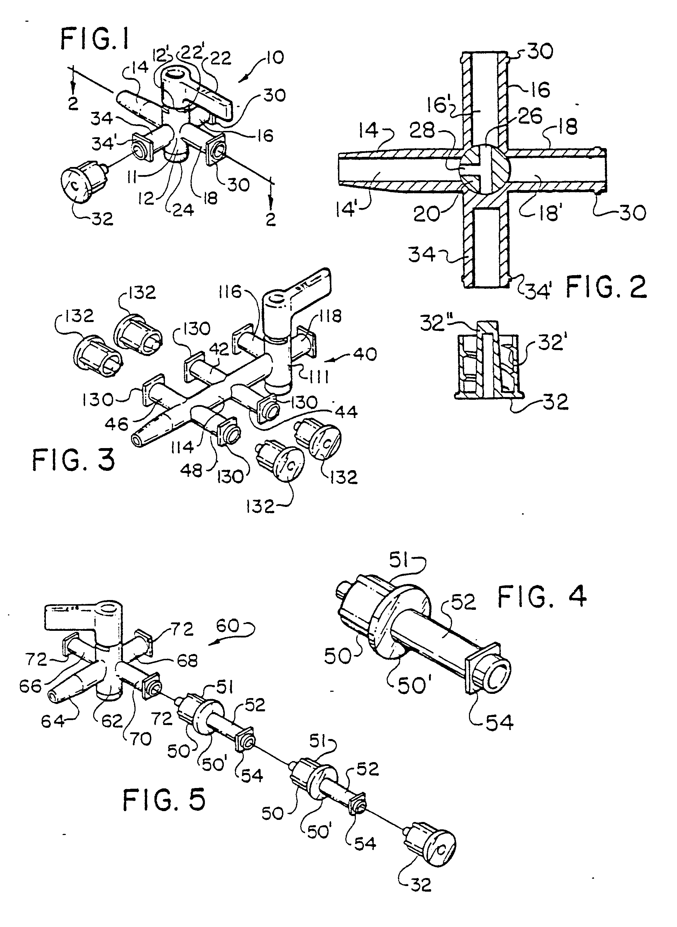

Referring now to the accompanying drawings and initially to Figures 1 and 2, one preferred embodiment of the stopcock valve assembly of the present invention is indicated generally at 10. The valve assembly 10 includes a stopcock valve 11 having a cylindrical valve body 12 from which tubular extensions 14, 16, 18 radially extend coplanarly at ninety degree spacings. The valve body 12 is hollow and each tubular extension 14, 16, 18, opens therein through a respective fluid port 14', 16', 18' in the valve body 12 for fluid communication between the tubular extensions 14, 16, 18 through the hollow interior of the valve body 12. A cylindrical core 20 of greater axial extent than the valve body 12 is rotatably fitted snugly in the valve body 12 with a portion of the core 20 extending upwardly from the upper edge of the valve body 12 and another portion of the core 20 extending downwardly from the lower edge of the valve body 12. A handle 22 is rigidly affixed to the upwardly extending core portion for actuating rotational movement of the core 20 within the valve body 12. The lowermost end of the core 20 has affixed thereto a cylindrical retaining member 24 of approximately the same diameter as the valve body 12. The core 20 has a bore 26 formed diametrically therethrough and another bore 28 formed radially therethrough perpendicularly to and opening into the bore 26, the bores 26, 28 being thusly formed at a selected disposition axially along the core 20 to be in the same plane as the ports 14', 16', 18' and tubular extensions 14, 16, 18 in the assembled form of the valve 11 to provide for selective communication between any two of the tubular extensions 14, 16, 18 by selective rotational positioning of the core 20 within the valve body 12. For convenience of operation, the handle 22 is affixed to the core 20 to extend radially oppositely of the bore 28 whereby in any operative position of the core 20 the handle 22 extends in the direction of the tubular extension 14, 16, or 18 which is closed by the core 20 in the respective operative position and cooperating shoulders 12', 22' are formed on the valve body 12 and the handle 22 as stop surfaces for accurate positioning of the handle 22. The tubular extension 14 is tapered in the form of a male taper so as to be adapted to fit into the female taper of a hypodermic needle hub (not shown) or to be connected to conventional flexible tubing (not shown). Each of the tubular extensions 16, 18 are formed at their respective extending ends with a hollow female fitting 30 in the form of a female Luer lock fitting having an outward annular flange, capable of receiving and engaging a mating cap 32 of the Luer type of lock with the projecting male engaging portion 32" of the cap 32 engaged in the tubular fitting 30 and the interior threads 32' of the cap 32 threadedly engaging the flange of the fitting 30, or alternatively capable of receiving conventional flexible tubing (not shown).

As will be understood, the stopcock assembly 10 as thus far described is of the conventional type described and illustrated in the US—A—3,185,179 and US—A—4,207,923 to which reference may be had for a more complete specification thereof. According to the first embodiment of the present invention, another tubular extension 34 is formed on and extends outwardly from the valve body 12 coplanarly with the tubular extensions 14, 16, 18 intermediate the tubular extensions 14, 18 but is inwardly closed to communication with the hollow interior of valve body 12 and therefore is inoperative for fluid communication through the valve body 12 with the ports 14', 16', 18' and tubes 14, 16, 18, the tubular extension 34 having formed thereon at its outward end a hollow flanged female Luer lock fitting 34' identical to the Luer fittings 30 whereby the tubular extension 34 constitutes in effect a blind extension for the accessory purpose of inserting therein and threadedly mounting thereon a Luer lock cap 32 for storage thereof when not in port-closing use on one of the tubular extensions 16, 18 and to provide ready access to the stored cap 32 for engagement on one of the tubular extensions 16, 18 when desired. Thus, a cap 32 may be conveniently stored for ready use in a manner shielding the male projecting portion 32" and the interior threads 32' of the cap 32 to best insure the sterility thereof. It is contemplated that ordinarily the blind tubular extension 34 will be provided with a cap 32 of its own in setting the valve 11 for use whereby the stored cap 32 will be readily accessible for use when or if necessary such as for replacement of a cap 32 removed from one of the tubular extensions 16, 18 during a lengthy sampling or similar procedure or for quick replacement of a removed cap 32 which has been lost or misplaced. As will be understood, the caps 32 are inexpensive and are considered disposable and therefore such above-described use of the blind tubular extension 34 is considered preferable insofar as insuring and best maintaining sterile conditions. Alternatively, the blind tubular extension 34 may be employed for temporarily mounting thereon for storage a cap 32 during removal thereof from one of the tubular extensions 16, 18 to provide ready access to the cap 32 for its replacement on the tubular extension 15, 18 when desired.

For example, in the infusion of blood or other fluids into the human body, stopcock assemblies 10 of the present type are routinely employed as a metering valve in the flexible tubing used in such a procedure. As will be understood, the stopcock 10 is typically arranged with the tubular extension 18 acting as an inlet conduit communicating through a length of tubing (not shown) with the fluid source, with the tubular extension 14 acting as an outlet conduit communicating through another length of tubing (not shown) with the patient, and with the core 20 positioned to align the bore 26 with the tubular extensions 14, 18 for communication therebetween, the tubular extension 16 thereby being closed and out of operation and the Luer lock cap 32 being fitted thereover. In this conventional infusion procedure, it is an ordinary control procedure to periodically divert a small quantity of the infusion fluid for testing as well to periodically withdraw from the patient a small quantity of body fluid for testing, this being done when desired by removing the Luer lock cap 32 from the tubular extension 16 and operating the stopcock handle 22 to provide communication between the tubular extension 16 and the tubular extension 18, in the first instance, and alternatively the tubular extension 14, in the second instance. In either case, the operation generally requires the attendant performing the procedure to use both hands and further may take several minutes to complete. As above mentioned, with conventional stopcocks, the Luer lock cap 32 must accordingly be set aside and is often misplaced temporarily or lost altogether. Since it is important to maintain sterile conditions, it is highly desirable to complete such a fluid sampling operation as quickly as possible and to minimize the time period during which the Luer lock cap 32 is removed from the tubular extension 16. Merely setting aside the cap 32 creates a possibility that it may be contaminated by bacteria, germs and the like and furthermore any misplacement, however temporary, or loss of the cap 32 greatly increases the chances of the introduction of bacteria, germs or the like into the stopcock 10 through the tubular extension 16. The provision in the present stopcock 10 of the blind tubular extension 34 eliminates these dangers by providing a convenient, non-interfering mated fitting on which a Luer lock cap 32 may be received and retained for storage thereof to provide ready access to the stored cap 32 when need such as in sampling or similar procedure of the above-described type. Once the procedure is completed, the cap 32 may be readily accessed for placement on the tubular extension 16. Thus, the storage of the cap 32 on the blind fitting 34 not only tends to maintain the cap 32 more sterile than if it were merely placed aside, but also helps to minimize any chance that germs, bacteria or the like may enter the valve 11 through the tubular extension 16.

Those skilled in the art will understand that the present invention is susceptible of a broad utility not limited to the above-described specific embodiment and particularly that the blind tubular extension 34 need not necessarily be formed at the described location on the valve body 12. Thus, the tubular extension 34 may for instance be formed on one of the tubular extensions 14, 16, 18 or on the retaining member 24 to extend downwardly therefrom, or on the handle 22 at its axis to extend upwardly therefrom. Furthermore, the blind tubular extension 34 may readily be embodied in types of medical stopcocks or valves other than the three-way type stopcock valve 11 and, if desirable, more than one blind tubular extension 34 may be provided. Additionally, it will be understood that the present invention is not limited in application to stopcock valves employing Luer-type lock caps 32 and female Luer-type lock fittings nor is it critical that the blind tubular extension 34 be formed with a female fitting of the Luer lock type 34', but instead the present invention may be readily adapted in any type stopcock valve employing a closure device on a mating out-of-use port and the blind tubular extension may accordingly be formed with any type fitting appropriate to accommodate the closure device in use. For instance, various types of conventional caps or other closure devices other than a Luer lock cap 32 may be employed in the particular embodiment of Figure 1, such as a plain, non-threaded cap having no male projecting portion, and, similarly various types of conventional female fittings other than the flanged-type Luer lock fitting 34' which are also adapted to receive a Luer lock cap 32 or other cap or closure device may be employed in the particular embodiment of Figure 1, such as a threaded screw-type, non-flanged fitting or a simply non-flanged, non-threaded cylindrical tubular extension both of which are equally capable of receiving and retaining a Luer lock cap 32. It is also contemplated that the blind portion or member may be constructed to substantially or entirely enclose sealably the interior of the stored Luer lock or other cap or to otherwise seal the valve engaging, port closing surfaces and portions of the closure device being employed thereby to maintain the stored cap or other closure device in a sterile condition prior to use thereof. Thus, for example, the blind portion may be formed to engage sealably the outer rim of the Luer lock cap 32. In addition, the blind tubular extension or extensions or the blind portion or portions may be constructed to be readily attachable and detachable from the valve body 12 for ready replacement of the blind portion or portions and one or more caps or closure devices carried thereby as a unit following closure caps or closure devices stored thereon.

One modified form of stopcock assembly is shown in Figure 3 at 40 and basically includes a stopcock valve portion 111 of the same three-way construction as the stopcock 11 having flanged tubular extensions 114, 116, 118 with its tubular extension 114 being substantially elongated and having formed therealong four blind accessory tubular extensions 42, 44, 46, 48 each being closed from operative communication with the interior of the tubular extension 114 and each having a hollow flanged female Luer lock fitting 130 at its respective extending end for threadedly receiving and storing a respective Luer lock cap 132. For convenience and flexibility of use of this form of the present invention with conventional stopcocks 11, it is contemplated that the elongated portion of tubular extension 114 may be constructed as a separate individual item adapted for assembled engagement with the male Luer taper of the tubular extension of a conventional stopcock 11 to form the valve assembly 40. Those skilled in the art will appreciate that blind tubular extensions according to the present invention may be equally well embodied in other forms of attachments adapted for engagement on conventional stopcock valves to facilitate storage of one or more Luer lock caps 32, such as, for example, a clip-type device formed to be friction snap-fitted on the valve body and having one or more female fittings 34 formed thereon for receiving caps 32.

Figure 4 illustrates another embodiment of the present invention in a modified form of a Luer lock cap, indicated generally at 50. According to this embodiment, the Luer lock cap 50 is formed with a cap portion 51 of the conventional construction of the caps 32, 132 having a cylindrical portion with a projecting male engaging portion and with interiorly formed threads (not shown) adapted for insertion in and to threadedly engage the flanges of a conventional female Luer lock fitting (e.g. 30, 130, Figures 1, 2, 4). Affixed to and extending outwardly from the flat radial surface 50' of the cap 50 is a blind tubular extension 52 inwardly closed from communication through the cap 50 which extension 52 has a hollow flanged end portion 54 of the form of a conventional female Luer lock fitting adapted to threadedly receive either a conventional Luer lock cap 32 or the cap portion 51 of another modified cap 50. In this manner, the Luer lock cap 50 provides substantial flexibility in the application and use of the present invention in that, employing the Luer lock cap 50, certain conventional stopcocks may be readily adapted to practice the present invention by merely mounting the Luer lock cap 50 on a non-operating tubular extension thereof and alternatively the Luer lock cap 50 may be mounted on the blind tubular extension of a stopcock 11 or 40 of the present invention and plural additional Luer lock caps 50 and/or a conventional cap 32 may be stacked on the first cap 50 (Figure 5) to increase the cap storage capacity of the stopcocks 11, 40.

More specifically, in the first above-described instance of use of the Luer lock cap 50, any conventional medical stopcock valve of at least a four-way or greater capability having four or more tubular extensions at least two of which have female Luer lock fittings may be readily adapted for use in the above-described three-way manner of the stopcocks 11, 40 or in any similar manner. As seen in Figure 5, a conventional stopcock valve of the four-way type is generally indicated at 60 and includes a valve body 62 having four fluid directing ports formed as tubular extensions 64, 66, 68, 70 from the valve body 62 and an appropriate interior control member (not shown) disposed in the valve body 62 to control selectively communication between the four ports. Each of the three tubular extensions 66, 68, 70 are formed at their outer ends with a flanged female Luer lock fitting 72 adapted conventionally to receive a Luer lock cap. The various modes of operation of the valve 60 are well known and need not be described herein, but reference may be made to U.S.—A—3,834,372 for a disclosure of one exemplary such valve 60. As will be understood, the valve 60 may be employed in either a two-way, three-way or four-way manner as desired and, in using it in a three-way manner, only three of the fluid-directing tubular extensions, e.g. 64, 66, 68 will be needed for operation and the other tubular extension 70 will be out of operation throughout the use of the valve 60. Accordingly, in such a use of the valve 60, the modified Luer lock cap 50 may be mounted on the fitting of the out-of-use tubular extension 70 to permit the stacked storage of plural modified Luer lock caps 50 as shown in Figure 5. As those skilled in the art will recognize, such a use of the modified Luer lock cap 50 to adapt conventional valves to the present invention will find greatest practical application in valves of greater than four-way capacity wherein only one tubular extension may not be needed for operation but the capacity for storage of several Luer lock caps at one time may be required. The particular embodiment of the present invention shown in Figure 5 is therefore considered to be illustrative of such a use of the cap 50 of the present invention and not to limit the scope thereof.

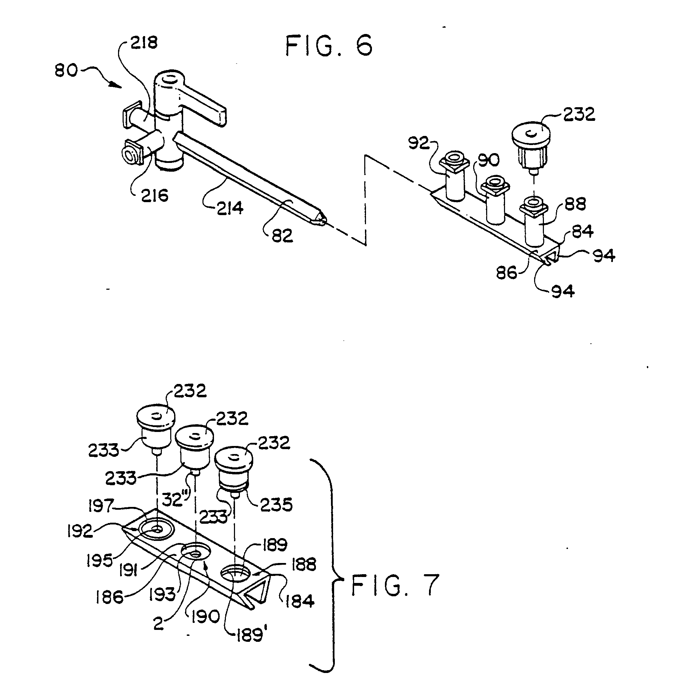

Figure 6 illustrates another modified form of three-way stopcock valve assembly indicated generally at 80 having tubular extensions 214, 216, 218 the tubular extension 214 of which is substantially elongated and on the exterior of which is formed a longitudinally extending track 82 of an inverted triangular shape. A cap storage member 84 capable of receiving and storing several Luer lock caps 232 is provided for selective attachment to and detachment from the track 82, the cap storage member 84 having a substantially flat platform 86 from one surface of which extend three blind accessory tubular extensions 88, 90, 92 of female Luer lock construction adapted to receive three Luer lock caps 232 and from the longitudinal side edges of which respective flanges 94 extend convergingly in the opposite direction from the extensions 88, 80, 92 at angles compatible with the track 82 for sliding attachment thereon and removal therefrom. It will thus be understood that the cap storage member 84 with three caps 232 stored thereon may be attached as a unit to the tubular extension 214 to provide ready access to the caps 232 for use as needed and then may be readily removed following usage of each of the caps 232 for ready replacement with another fully stocked cap storage member 84 and for replenishment of the original cap storage member 84 with fresh sterile caps. In this manner, a sufficient supply of caps 232 may always be maintained readily available for use with minimal time being necessary for replacing used caps 232 with fresh stored caps 232.

Another cap storage method member 184 of the type of cap storage member 84 is illustrated in Figure 7 and is provided with three alternative arrangements for securing mounting of the caps 232 in a totally sealed manner, indicated generally at 188, 190, 192. As will be understood, the several cap mounting arrangements in any one cap storage member of this type will ordinarily be of the same construction, the cap storage member 184 having three differing forms of cap mounting arrangement solely for purposes of illustration. With the conventional male-female Luer lock mounting of the foregoing described constructions, substantially only the projecting male engaging portion 32″ is sealably shielded during storage and therefore some risk exists that the remaining interior surfaces of the cap such as the interior threads 32′ may become contaminated during storage of the cap and may transmit the contamination to the valve upon later use of the stored cap. The cap mounting arrangements 188, 190, 192 substantially eliminate this risk by sealably engaging in alternate manners the outer rim 233 of a stored cap 232 thereby entirely enclosing and shielding the interior of the cap 232 to maintain its sterile. More specifically, the cap mounting arrangement 188 provides a cylindrical recess 189 in the platform 186 of the cap storage member 184 the side wall of which recess 189 is threaded at 189′ to be adapted for sealed threaded engagement with compatible threads 235 specially formed exteriorly on a modified cap 232 adjacent its outer rim 233. The cap mounting arrangement 190 provides a first cylindrical recess 191 adapted to receive the outer rim 233 of a cap 232 by a relatively close sealed friction fit with further central recess 193 therein adapted to receive the projecting male engaging portion 32″. The cap mounting arrangement 192 provides only a small central recess 195 adapted to receive the male engaging portion 32″ of a cap 232 and has a circular adhesive gasket 197 on the surface of the platform 186 for sealing engagement of the outer rim 233 of the cap 232.

Those persons skilled in the art will readily recognize that other cap mounting arrangements are equally adaptable for sealed engagement with the outer rim of a stored cap within the scope of the present invention. For instance, the outer cap rim and the blind portion may be compatibly tapered to frictionally fit together in a substantially similar manner to the tapered engagement between the male engaging portion and female receiving portion of a conventional Luer lock system.

The present invention has been described in detail above for purposes of illustration only and is not intended to be limited by this description or otherwise to exclude any variation or equivalent arrangement that would be apparent from, or reasonably suggested by the foregoing disclosure to the skill of the art.

**Claims**

1. A fluid conveying member (10, 40, 60, 80) for use in a medical fluid flow system or the like comprising a tubular portion (14, 16, 18, 114, 116, 118, 64, 66, 68, 70, 214, 216, 218) having a fluid-conveying passageway therethrough, said tubular portion having connection means at the ends of its said fluid-conveying passageway for engagement with other fluid conveying members in fluid communication therewith, at least one closure means (32, 132, 232) of the type having engagement means (32′, 32″) formed for selective port-closing engagement with a medical stopcock valve assembly, characterized by at least one blind closure-receiving means (34, 42, 44, 46, 48, 52, 88, 90, 92, 188, 190, 192) on said tubular portion out of operative communication with said fluid passageway and having a portion (34′, 130, 54) formed for engagement by a stored closure means having engagement means identical to said at least one closure means to receive and retain said stored closure means for storage thereof when not in port-closing use to prevent loss thereof and to provide ready access thereto for tube-closing use when desired.

2. A fluid conveying member according to claim 1 and characterized further in that said at least one closure means (32, 132, 232) constitutes said stored closure means to be received and retained on said blind closure-receiving means (34, 42, 44, 46, 48, 52, 88, 90, 92, 188, 190, 192).

3. A fluid conveying member according to claim 1 and characterized further in that said stored closure means comprises a cap (32, 132, 232), said engagement means thereof including a projecting male engaging portion (32″) formed thereon for engagement in a mating female member (16, 18, 34, 116, 118, 42, 44, 46, 48, 66, 68, 70, 216, 218, 88, 90, 92), and said blind closure-receiving means is formed as a tubular female fitting (34, 42, 44, 46, 48, 52, 88, 90, 92) adapted for mating receiving engagement with said projecting portion of said stored closure means.

4. A fluid conveying member according to claim 1 and characterized further in that said blind closure-receiving means (188, 190, 192) is adapted for sealing said engagement means (32′, 32″) of said stored closure means (232) for maintaining said engagement means sterile.

5. A fluid conveying member according to claim 4 and characterized further in that said stored closure means comprises a cap (232) having an outer rim (233) and having said engagement means (32′, 32″) formed interiorly, said blind closure-receiving member including means (189′, 191, 197) for sealably engaging said rim for maintaining said engagement means sterile.

6. A fluid conveying member according to claim 5 and characterized further in that said sealably engaging means includes means (189′, 191) for frictionally engaging snugly said rim (233).

7. A fluid conveying member according to claim 6 and characterized further in that said rim (233) and said frictionally engaging means (191) respectively have mating tapered surfaces for engagement therebetween.

8. A fluid conveying member according to claim 1 and characterized further by a closure storage unit (84, 184) having plural blind closure-receiving means (88, 90, 92, 188, 190, 192) each formed for engagement by a stored closure means (232) said unit (84, 184) being removably attached to said tubular portion (214) for ready removal following use of said plural stored closure means and replacement thereof with another unit of plural blind closure-receiving means carrying plural stored closure means for use thereof.

9. A fluid conveying member according to claim 8 and characterized further in that said closure storage unit (84, 184) and said tubular portion (214) having mated affixation means (82, 94) formed thereon for said sliding receipt of said closure storage unit on said tubular portion for ready access to said stored closure means for port closing use for ready sliding removal of said closure storage unit from said tubular portion following use of said stored closure means for replenishment with other closure means or replacement by another closure storage member with other closure means stored thereon.

10. A fluid conveying member according to claim 1 wherein said fluid conveying member comprises a medical stopcock valve assembly (10, 40) having a hollow valve body (11, 111) having a plurality of fluid directing tubes (14, 16, 18, 114, 116, 118) communicating interiorly with and projecting from said valve body, each of at least one of said tubes having a tubular female fitting (30), a valve operating member (20) movably disposed interiorly of said valve body for selective movement to control fluid communication through said valve body between said fluid directing tubes, and cap means (32, 132) having a projecting male engaging portion (32″) formed thereon for selective engagement in the tubular female fitting for closure of its said fluid-directing tube, and characterized further by a blind accessory tube (34, 42, 44, 46, 48) formed integrally on said valve body closed to operative communication interiorly with said valve body and said tubes and including a female fitting (34′, 130) formed for mated receiving engagement of said projecting portion of said cap means.

11. A fluid conveying member according to claim 1 and characterized further by plural said blind closure-receiving means (42, 44, 46, 48) on said valve body for receiving and retaining plural said stored closure means (132) for ready access to each thereof for use.

## Patentansprüche

1. Ein Flüssigkeit leitendes Bauteil (10, 40, 60, 80) zur Verwendung in einem medizinischen Flüssigkeits-fließsystem oder ähnlichen, bestehend aus einem rohrförmigen Abschnitt (14, 16, 18, 114, 116, 118, 64, 66, 68, 70, 214, 216, 218) mit einem dort hindurchgehenden Flüssigkeit leitenden Durchlaß, wobei der rohrförmige Abschnitt an den Enden des Flüssigkeit leitenden Durchlasses Anschlußmittel zum Eingriff mit anderen Flüssigkeit leitenden Bauteilen in Flüssigkeitsverbindung damit aufweist, mit mindestens einem Verschlußteil (32, 132, 232) des Eingriffsmittel (32′, 32″) aufweisenden Typs, die ausgebildet sind zum wahlweisen öffnungsverschließenden Eingriff mit einer medizinischen Absperrventilbaugruppe, gekennzeichnet durch mindestens ein blindes, verschlußaufnehmendes Teil (34, 42, 44, 46, 48, 52, 88, 90, 92, 188, 190, 192) an dem rohrförmigen Abschnitt außerhalb einer operativen Verbindung mit dem Flüssigkeitsdurchlaß, das einen Abschnitt (34′, 130, 54) aufweist, der zum Eingriff mit einem bevorrateten Verschlußteil ausgebildet ist, das Eingriffsmittel aufweist, die identisch zu dem mindestens einen Verschlußteil sind, um das bevorratete Verschlußteil zur Lagerung darauf aufzunehmen und festzuhalten, wenn es nicht im öffnungsverschließenden Gebrauch ist, um seinen Verlust zu verhindern und um es griffbereit für einen rohrverschließenden Gebrauch, wenn gewünscht, zu halten.

2. Ein Flüssigkeit leitendes Bauteil nach Anspruch 1, und weiterhin gekennzeichnet, daß das mindestens eine Verschlußteil (32, 132, 232) das bevorratete Verschlußteil bildet, das von dem blinden, verschlußaufnehmenden Teil (34, 42, 44, 46, 48, 52, 88, 90, 92, 188, 190, 192) aufzunehmen und festzuhalten ist.

3. Ein Flüssigkeit leitendes Bauteil nach Anspruch 1 und weiterhin gekennzeichnet, daß das bevorratete Verschlußteil aus einer Kappe (32, 132, 232) besteht, deren Eingriffsmittel einen daran angeformten, vorstehenden, männlichen Eingriffsteil (32′) zum Eingriff in ein dazu passendes weibliches Teil (16, 18, 34, 116, 118, 42, 44, 46, 48, 66, 68, 70, 216, 218, 88, 90, 92) umfaßt und das blinde verschlußaufnehmende Teil als ein rohrförmiger weiblicher fitting (34, 42, 44, 46, 48, 52, 88, 90, 92) ausgebildet ist, der zum passenden Aufnahmeeingriff mit dem vorstehenden Teil des bevorrateten Verschlußteils angepaßt ist.

4. Ein Flüssigkeit leitendes Bauteil nach Anspruch 1 und weiterhin gekennzeichnet, daß das blinde, verschlußaufnehmende Teil (188, 190, 192) zur Abdichtung des Eingriffsmittels (32′, 32″) des bevorrateten Verschlußteils angepaßt ist, um das Eingriffsmittel steril zu halten.

5. Ein Flüssigkeit leitendes Bauteil nach Anspruch 4 und weiterhin gekennzeichnet, daß das bevorratete Verschlußteil aus einer Kappe (232) besteht, die einen äußeren Rand (233) und das Eingriffsmittel (32′, 32″) im Inneren angeformt aufweist, wobei das blinde, verschlußaufnehmende Teil Mittel (189′, 191, 197) zum dichten Eingriff des Randes umfaßt, um das Eingriffsmittel (32′, 32″) steril zu halten.

6. Ein Flüssigkeit leitendes Bauteil nach Anspruch 5 und weiterhin gekennzeichnet, daß das dichte Eingriffsmittel Mittel (189′, 191) zum reibschlüssigen, satt anliegenden Eingriff des Randes (233) umfaßt.

7. Ein Flüssigkeit leitendes Bauteil nach Anspruch 6 und weiterhin gekennzeichnet, daß der Rand (233) und das reibschlüssige Eingriffsmittel (191) jeweils zum gegenseitigen Eingriff passende kegelförmige Oberflächen aufweisen.

8. Ein Flüssigkeit leitendes Bauteil nach Anspruch 1 und weiterhin gekennzeichnet, durch eine Verschlußbevorratungseinheit (84, 184), mit einer Vielzahl von blinden, verschlußaufnehmenden Teilen (88, 90, 92, 188, 190, 192), welche jeweils zum Eingriff durch ein bevorratetes Verschlußteil (232) ausgebildet sind, diese Einheit (84, 184) lösbar an dem rohrförmigen Abschnitt (214) befestigt ist, zum jederzeitigen Entfernen nach dem Gebrauch der Vielzahl von bevorrateten Verschlußteilen und dem Ersatz dieser durch eine andere Einheit von eine Vielzahl aufweisenden, blinden verschlußaufnehmenden Teilen, die eine Vielzahl von bevorrateten Verschlußteilen zu deren Verwendung tragen.

9. Ein Flüssigkeit leitendes Bauteil nach Anspruch 8 und weiterhin gekennzeichnet, daß die Verschlußbevorratungseinheit (84, 184) und der rohrförmige Abschnitt (214) passende daran angeformte Befestigungsmittel (82, 84) zur gleitenden Aufnahme der Verschlußbevorratungseinheit auf dem rohrförmigen Abschnitt aufweist, um die bevorrateten Verschlußteile zum öffnungsverschließenden Gebrauch griffbereit zu halten und um jederzeit die Verschlußbevorratungseinheit vom rohrförmigen Abschnitt nach dem Gebrauch der bevorrateten Verschlußteile gleitend entfernen zu können, zum Nachfüllen mit anderen Verschlußteilen oder dem Austausch durch ein anderes Verschlußbevorratungsteil mit darauf bevorrateten anderen Verschlußteilen.

10. Ein Flüssigkeit leitendes Bauteil nach Anspruch 1, bei dem das Flüssigkeit leitende Bauteil aus einer medizinischen Absperrventilbaugruppe (10, 40) besteht, die einen hohlen Ventilkörper (11, 111) mit einer Vielzahl von Flüssigkeit leitenden Rohren (14, 16, 18, 114, 116, 118) aufweist, die im Inneren mit dem Ventilkörper kommunizieren und von diesem abstehen, mindestens eines der Rohre einen rohrförmigen weiblichen fitting (30) aufweist, ein Ventilbetätingungsteil (20) beweglich im Inneren des Ventilkörpers angeordnet ist, um durch wahlweise Bewegung die Flüssigkeitsverbindung durch den Ventilkörper zwischen den Flüssigkeit leitenden Rohren zu steuern, und ein Kappenteil (32, 132) einen daran angeformten vorspringenden männlichen Eingriffsteil (32″) zum wahlweisen Eingriff in den rohrförmigen weiblichen fitting zum Verschließen seines Flüssigkeit leitenden Rohres aufweist und weiterhin gekennzeichnet durch ein einstückig am Ventilkörper angeformtes blindes Zusatzrohr (34, 42, 44, 46, 48), das zur Verhinderung einer operativen Verbindung mit dem Ventilkörper und den Rohren innen verschlossen ist und daran angeformt einen weiblichen fitting (34′, 130) zum passenden Aufnahmeeingriff des vorstehenden Teils des Kappenteils umfaßt.

11. Ein Flüssigkeit leitendes Bauteil nach Anspruch 1 und weiterhin gekennzeichnet durch eine Vielzahl von blinden verschlußaufnehmenden Teilen (42, 44, 46, 48) auf dem Ventilkörper zur Aufnahme und zum Festhalten der bevorrateten Verschlußteile (132) für den jeweiligen griffbereiten Halt für den Gebrauch.

**Revendications**

1. Un dispositif de transfert du liquide (10, 40, 60, 80) pour utiliser dans un circuit de liquide médical ou similaire comprenant une partie tubulaire (14, 16, 18, 114, 116, 118, 64, 66, 68, 70, 214, 216, 218) ayant un passage conducteur de liquide, ladite partie tubulaire est munie d'un dispositif de raccord aux extrémités dudit passage conducteur de liquide afin d'assurer la liaison avec les autres dispositifs du transfert de système communiquant, au moins un dispositif de fermeture (32, 132, 232) du type muni d'un dispositif de liaison (32′, 32″) embouti avec un ensemble robinet médical d'arrêt à soupape pour la fermeture sélective, caractérisé par au moins un récepteur d'obturateur (34, 42, 44, 46, 48, 52, 88, 90, 92, 188, 190, 192) sur la partie tubulaire hors du contact opératoire avec ledit passage conducteur de liquide ayant une partie (34′, 130, 54) destinée à être connectée au dispositif de stockage des obturateurs muni d'un raccord identique au dudit, au moins un dispositif de fermeture pour la réception, la rétention, et le stockage des obturateur lorsque non utilisés en tant que fermetures sur le circuit évitant de ce fait leur perte et assurant l'accés immédiat pour la fermeture du tuyau si désiré.

2. Dispositif de transfert du liquide selon la revendication 1 et caractérisé par la suite en ce que au moins un dispositif de fermeture (32, 132, 232) se trouve en stock reçu et retenu par ledit récepteur de l'obturateur (34, 42, 44, 46, 48, 52, 88, 90, 92, 188, 190, 192).

3. Un dispositif du transfert de liquide selon la revendication 1 caractérisé par la suite par ledit dispositif de fermeture stocke contient un bouchon (32, 132, 232), ce dispositif contient un raccord mâle saillant (32″) destiné à être engagé dans une pièce d'assemblage femelle (16, 18, 34, 116, 118, 42, 44, 46, 48, 66, 68, 70, 216, 218, 88, 90, 92) et ledit récepteur d'obturateur représente un raccord femelle tubulaire (34, 42, 44, 46, 48, 52, 88, 90, 92) adapté pour la réception de ladite partie saillante du dispositif de fermeture (obturateur) stocké.

4. Un dispositif du transfert de liquide selon la revendication 1 et se caractérisé par la suite en ce que ledit récepteur de l'obturateur (188, 190, 192) est adapté pour sceller ledit raccord (32′, 32″) de ladite fermeture stocké (232) de manière à garder ce raccord stérile.

5. Le dispositif du transfert de liquide selon la revendication 4 et caractérisé par la suite en ce que ledit dispositif de fermetures emmagasinées comprend un bouchon (232) muni d'un bourrelet extérieur (233) avec ledit raccord (32′, 32″) situé à l'intérieur. Ledit récepteur d'obturateur possède un dispositif (189', 191, 197) qui permet une obturation hermétique par ce bourrelet gardant ainsi le raccord stérile.

6. Le dispositif du transfert de liquide selon la revendication 5 et caractérisé par la suite en ce que ledit raccord scellable comprend un dispositif (189', 191) qui par friction scelle convenablement ledit bourrelet (233).

7. Le dispositif du transfert de liquide selon la revendication 6 et caractérisé par la suite en ce que ledit bourrelet (233) et ledit dispositif d'obturation par friction (191) ont respectivement des surfaces obliques de contact ce qui leur permet de se raccorder mutuellement.

8. Le dispositif du transfert de liquide selon la revendication 1 et caractérisé par la suite en ce que le magasin des fermetures (84, 184) est muni de plusieurs récepteurs d'obturateurs (88, 90, 92, 188, 190, 192) chacun destiné à recevoir un dispositif de la fermeture (232) ce magasin (84, 184) est attaché à la partie tubulaire (214) de manière à permettre un détachement facile après usage du magasin et son remplacement par une autre unité de récepteurs d'obturateurs comportant plusieurs fermetures.

9. Le dispositif du transfert de liquide selon la revendication 8 et caractérisé par la suite en ce que ledit magasin de fermetures (84, 184) et ladite partie tubulaire (214) ont des surfaces d'assemblage (82, 94) destinées à recevoir par glissement ledit magasin sur la partie tubulaire et ceci pour faciliter l'accès aux dispositifs emmagasinés de fermetures d'orifices et pour un enlèvement aisé dudit magasin de cette partie tubulaire après utilisation desdites fermetures pour remplir à nouveau ce magasin avec d'autres fermetures ou le remplacer par au autre déja rempli.

10. Le dispositif du transfert de liquide selon la revendication 1 ledit dispositif contient l'ensemble du robinet médical d'arrêt (10, 40) le corps creux du robinet (11, 111) est muni de plusieurs tubes conducteurs de liquide (14, 16, 18, 114, 116, 118) qui communiquent entre eux intérieurement et saillent dudit corps, chacun ou au moins un se ces tubes est muni raccord tubulaire femelle (30), dispositif mobile (20) actionné par soupape se trouve à l'intérieur dudit corps de robinet il contrôle par son mouvement sélectif — le transfert du liquide entre lesdits tubes conducteurs à travers le corps du robinet et les bouchons (32, 132) avec le raccord mâle (32") saillant destiné à être engagé dans un raccord femelle tubulaire pour la fermeture dudit tube de liquide correspondant, et caractérisé ensuite par un tube accessoire obturé (34, 42, 44, 46, 48) situé intégralement sur ledit corps de robinet près de la voie communiquente intérieure entre ledit corps de robinet et les tubes et ils comprennent un raccord femelle (34', 130) destiné à être assemblé avec la partie saillante de 1' obturateur.

11. Le dispositif du transfert de liquide selon la revendication 1 et caractérisé en suite par plusieurs récepteurs d'obturateurs (42, 44, 46, 48) sur le corps du robinet pour la réception et le maintien desdits dispositifs de fermeture emmagasinés (132) assurant ainsi la facilité d'accès à chacun d'entre eux et leur utilisation.

FIG. 1

FIG. 2

FIG. 3

FIG. 4

FIG. 5

1

FIG. 6

FIG. 7